# EUROPEAN PATENT SPECIFICATION

(11) **EP 3 829 528 B1**
(45) Date of publication and mention of the grant of the patent: **20.03.2024**
(21) Application number: 19736734.5
(22) Date of filing: 10.07.2019
(51) Int. Cl.: A61K 8/31, A61K 8/36, A61K 8/44, A61K 8/46, A61Q 5/02, A61Q 5/12, A61K 8/73, A61K 8/81, A61Q 19/10, A61K 8/92, A61K 8/02

(54) **ENHANCED MOISTURIZER DEPOSITION IN CLEANSING LIQUIDS CONTAINING HYDROPHOBICALLY OR NON-HYDROPHOBICALLY MODIFIED ANIONIC POLYMERS**
ERHÖHTE BEFEUCHTUNGSMITTELABSCHEIDUNG IN REINIGUNGSFLÜSSIGKEITEN MIT HYDROPHOB ODER NICHTHYDROPHOB MODIFIZIERTEN ANIONISCHEN POLYMEREN
DÉPÔT D'HYDRATANT AMÉLIORÉ DANS DES LIQUIDES DE NETTOYAGE CONTENANT DES POLYMÈRES ANIONIQUES MODIFIÉS DE MANIÈRE HYDROPHOBE OU NON HYDROPHOBE

(30) Priority: 30.07.2018 EP 18186321
(43) Date of publication of application: 09.06.2021
(73) Proprietor: Unilever Global IP Limited, Wirral, Merseyside CH62 4ZD (GB); Unilever IP Holdings B.V., 3013 AL Rotterdam (NL)
(72) Inventor: DOUGHERTY, Lindsay, Kaitlin, New Haven, Connecticut 06519 (US); MILLER, Jamie, Lynn, Trumbull, Connecticut 06611 (US); VASUDEVAN, Tirucherai, Varahan, Trumbull, Connecticut 06611 (US)
(74) Representative: Fijnvandraat, Arnoldus
(86) International application number: PCT/EP2019/068536
(87) International publication number: WO 2020/025275

(56) References cited:
- WO-A1-2015/181789
- US-B2- 7 674 848
- US-B2- 8 158 566
- US-B2- 9 901 524
- Anonymous: "Personal Care ACULYN(TM) 38 Rheology Modifier", , 31 December 2007 (2007-12-31), XP055539544, Retrieved from the Internet: URL:https://www.dow.com/assets/attachments /business/pcare/aculyn/aculyn_38/tds/aculy n_38.pdf [retrieved on 2019-01-09]
- M. LIU ET AL: "Modified Corneosurfametry as a new accelerated high-throughput ex vivo methodology for predicting cleanser effects towards human skin", INTERNATIONAL JOURNAL OF COSMETIC SCIENCE., vol. 38, no. 2, 1 April 2016 (2016-04-01), pages 178-186, XP055539392, NL ISSN: 0142-5463, DOI: 10.1111/ics.12273

## Description

### Field of the invention

The invention relates to body and hair cleansing compositions which are lamellar structured and which contain oil-based moisturizers such as petrolatum and triglyceride oils (hereinafter "moisturizers"). Such moisturizers are typically added to cleansing compositions as neat materials or in a pre-emulsified form. Compositions of the invention also contain cationic deposition polymers used typically to enhance deposition of the moisturizers. Such cationic deposition polymers will typically interact with anionic structuring polymers, also typically used in such lamellar structured moisturizing compositions; such interaction between cationic deposition polymers ("CDP") and anionic structuring polymers reduces deposition efficiency of the CDP such that less moisturizer is deposited.

### Background of the invention

Skin and hair cleansing formulations that are moisturizing and also less damaging to skin and hair are highly desired by consumers. Lamellar cleansing compositions that are formulated with mild surfactants meet the above criteria as lamellar structures provide lotion like consistency that cues moisturization, and mild surfactants cause less damage to skin and hair. Additionally, unlike isotropic formulations, lamellar formulations can also hold large amounts of hydrophobic emollients such as triglyceride oils and petrolatum that provide clinical moisturization benefits. The deposition of these moisturizers is promoted by the addition of cationic deposition polymers such as guar hydroxypropyl trimonium chloride (US 5,085,857).

Lamellar formulations that contain high loading of emollient oils (moisturizers) have been exemplified in several patents assigned to Puvvada et al. See, for example, US 5,952,286, US 5,962,395, US 6,150,312 and US 6,426,326. However, none of these references disclose use of anionic polymeric structurants, hydrophobically modified or non-hydrophobically modified, to stabilize sub-micron sized moisturizer droplets that can be deposited efficiently in lamellar liquids comprising cationic deposition polymers.

US Patents 8,105,996 and 8,158,566 to Wei et al., teach the use of hydrophobically modified anionic polymers in lamellar formulations containing moisturizers (e.g., emollient oils). However, lamellar formulations exemplified in these references are structured with a significant amount of non-ionic emulsifiers.

They further require use of high levels of salt to form the lamellar structures. By contrast, our compositions have levels of 0.4%, preferably 0.35% and below of nonionic emulsifier of HLB from 1.4 to 13 Further, the high salts seen in compositions of Wei are also not desirable in formulations of our invention as they can cause corrosion to equipment during manufacturing. Our formulations, in contrast, are formulated with 2.75%, preferably 2.7% of sodium chloride or less. Additionally, there is no recognition in these Wei references how the structuring polymers affect deposition of moisturizers; and also of what role does the mildness of the formulation play in the deposition of such moisturizers. Compositions of our invention have a defined mildness value (measured by CIM value using Corneosurfametry), a methodology defined later.

As indicated above, cationic deposition polymers are typically used to promote deposition of oil based moisturizers in lamellar liquids. As also indicated, anionic polymers are used to structure the liquids, but these typically interact with the cationic deposition polymers to lower deposition.

US 9 901 524 B2 to L'Oréal relates to stable moisturizing cleansing compositions for skin and hair, capable of forming a lamellar phase with good foaming performances. The document teaches that this can be achieved by compositions comprising an aqueous phase, a non-volatile hydrocarbon oil, a glycinate type anionic surfactant, an amphoteric or zwitterionic surfactant and a saturated fatty acid. Compositions comprising guar hydroxypropyl trimonium chloride are not disclosed.

### Summary of the invention

Unexpectedly, applicants have found that specific types of structuring polymers, more particularly anionic structuring polymers especially particular cross-linked hydrophobically or non-hydrophobically modified anionic polymers, interfere minimally in deposition of sub-micron sized moisturizer droplets by cationic deposition polymer, while providing excellent stability.

More specifically, applicants have found that the hydrophobically or non-hydrophobically modified anionic polymer of the invention can be defined by a combination of (a) viscosity measured in 2% polymer solution at specified pH level; and (b) slope of viscosity of the shear rate curve of the polymer solution. While not wishing to be bound by theory, these variables are believed to be linked to the anionicity and the molecular weight of the polymers. When polymers fall within ranges defined by the values (a) and (b) noted above, enhanced moisturizer deposition is found in the presence of cationic deposition polymers.

The effect is further influenced by mildness of formulation (formulations have corneosurfametry Calorimetric Index of Mildness, CIM, greater than 53, preferably greater than 55). As also noted above, compositions of the invention comprise 0.4% or less, preferably 0.3% or less, more preferably 0.2% or less and most preferably 0.1% or less of non-ionic emulsifier of HLB 1.4-13 while maintaining stability of lamellar compositions. By contrast, compositions of Wei require greater than this amount of emulsifier to maintain lamellar stability (see Table 6 at end). Compositions of our invention also preferably comprise 2.7% or less, preferably 2.5% or less, more preferably 2% or less, e.g. 0.1 to 2% by wt salt.

The composition of the invention is defined as follows.

They are lamellar structured cleansers with mild surfactant systems that comprise:
a) 1 to 70%, preferably 2 to 30% and more preferably 5 to 20% of a mixture of anionic surfactant comprising acylglutamate, acylglycinate, acyl isethionate and/or methylacyltaurate; amphoteric comprising amphoacetate and /or zwitterionic surfactants comprising cocoamidopropyl betaine and/or sulphobetaine; as well as optional nonionic and other surfactants;
b) 0.1 to 20%, preferably 1 to 10% and more preferably 2 to 5 wt. % of a C₈ to C₁₄ fatty acid, such as caprylic acid, capric acid, lauric acid, myristic acid or mixtures thereof;
c) 0.1 to 25%, preferably 1 to 15% and more preferably 2 to 10% of a moisturizing, conditioning agent, such as emulsion of petrolatum and/ or triglyceride oil;
d) 0.01 to 5%, preferably 0.1 to 2% and more preferably 0.2 to 1% of a cationic deposition polymer (also may be referred to a cationic conditioning polymer) comprising guar hydroxvpropyl trimonium chloride; and,
e) 0.01 to 5%, preferably 0.05 to 2% or 0.1 to 1% of a hydrophobically or non-hydrophobically modified anionic cross linked polymer, with the said polymer(s) having a viscosity of 2% solution, when measured at pH 6.35 and shear rate of 5s⁻¹, in the range of 1 to 100 mPa.s (centipoise), more preferably 10 to 80 mPa.s (centipoise) and most preferably in the range of 20 to 60 mPa.s (centipoise); and
f) ) 0.1 to 20% by wt. lamellar structurant wherein the slope of the viscosity shear rate curve is in the range of - 0.6 to -1.2, more preferably in the range of - 0.7 to - 1.1 and most preferably in the range of - 0.8 to - 1.0; and
wherein the cleansing formulation comprises 0.4% or less, preferably 0.3% or less, more preferably 0.2% or less and most preferably 0.1% or less of nonionic emulsifiers of HLB 1.4 to 13, and 2.7% or less, preferably 2.5% or less and more preferably 2% or less of salt, especially sodium chloride, and preferably has a corneosurfametry Calorimetric Index of Mildness, CIM, of 53 or greater, preferably 55 or greater and more preferably 55 to 65, the composition being substantially free of sulfate-containing surfactant.

### Detailed description of the invention

Except in the examples, or where otherwise explicitly indicated, all numbers in this description indicating amounts of material or conditions of reaction, physical properties of materials and/or use are to be understood as modified by the word "about." As used throughout, ranges are used as shorthand for describing each and every value that is within the range. Any value within the range can be selected as terminus of the range. The use of "and/or" indicates that any one from the list can be chosen individually, or any combination from the list can be chosen.

For the avoidance of doubt, the word "comprising" is intended to mean "including" but not necessarily "consisting of" or "composed of." In other words, the listed steps or options need not be exhaustive.

Unless indicated otherwise, all percentages for amount or amounts of ingredients used are to be understood to be percentages by weight based on the active weight of the material in the total weight of the composition, which total is 100%. (see page 15)

The compositions of the invention are lamellar structured liquid cleansing compositions that comprise moisturizing conditioning agent, also referred to as moisturizers (e.g., petrolatum, triglyceride); and further comprise cationic deposition polymers.

In the presence of anionic thickening polymers, the cationic deposition polymers will typically interact with such thickener, and this interaction will negatively impact deposition of the moisturizer.

Unexpectedly, applicants have discovered that, when specific hydrophobically or non-hydrophobically modified anionic cross-linked thickening polymers are selected (defined both by viscosity at defined conditions, and slope of the viscosity shear rate curve), deposition of moisturizer is significantly enhanced relative to use of anionic structuring polymers generally. Quite surprisingly, the effect of enhanced deposition is further strengthened by the mildness of the composition as measured by CIM. Further, compositions of the invention use 0.4% or less, preferably 0.3% or less, more preferably 0.2% or less and most preferably 0.1% or less of non-ionic emulsifiers of HLB 1.4 to 13 and 2.7% or less, preferably 2.5% or less and more preferably 2% or less of salt, especially sodium chloride, while maintaining stability of lamellar composition. Higher levels of salts can be corrosive to machinery used in preparation of the composition.

More particularly, compositions of the invention comprise:
a) 1 to 70%, preferably 2 to 30% and more preferably 5 to 20% of (a) at least one anionic surfactant comprising acylglutamate, acylglycinate, acyl isethionate and/or methylacyltaurate; (b) at least one amphoteric comprising amphoacetate and /or zwitterionic surfactants comprising cocoamidopropyl betaine and/or sulphobetaine; and (c) optionally one or more nonionic surfactants, cationic surfactants or blends thereof;
b) 0.1 to 20%, preferably 1 to 10% and more preferably 2 to 5 wt. % of a structuring agent forming a lamellar phase; this may be, for example, a C₈ to C₁₄ fatty acid, such as lauric acid, myristic acid or mixtures thereof;
c) 0.1 to 25%, preferably 1 to 15% and more preferably 2 to 10% of a moisturizing, conditioning agent (oil or emollient), for example, emulsion of petrolatum and/ or triglyceride oil;
d) 0.01 to 5%, preferably 0.1 to 2% and more preferably 0.2 to 1% of a cationic deposition polymer (also known as cationic conditioner) comprising guar hvdroxypropyl trimonium chloride; and
e) 0.01 to 5%, preferably 0.05 to 2% or 0.1 to 1% of a hydrophobically or non-hydrophobically modified anionic cross linked polymer, preferably a cross linked polymer, with the said polymer(s) having a viscosity of 2% solution, when measured at pH 6.35 and shear rate of 5s⁻¹, in the range of 1 to 100 mPa.s (centipoise), more preferably 10 to 80 mPa.s (centipoise) and most preferably in the range of 20 to 60 mPa.s (centipoise); wherein the slope of the viscosity shear rate curve in the range of - 0.6 to -1.2, more preferably in the range of - 0.7 to - 1.1 and most preferably in the range of - 0.8 to - 1.0; and
f) 0.1 to 20% by wt. lamellar structurant,
wherein the cleansing formulation comprises 0.4% or less, preferably 0.1 to 0.35% nonionic emulsifiers of HLB 1.4 to 13; and 2.7% or less, preferably 2.5% or less and more preferably 2% or less of salt, especially sodium chloride, and preferably has a corneosurfametry Calorimetric Index of Mildness, CIM, of 53 or greater, preferably 55 or greater and more preferably 55 to 65, the composition being substantially free of sulfate-containing surfactant.

The compositions are described more particularly below:

### Surfactants

The surfactant system of the subject invention comprises 1 to 50% by weight, preferably 2 to 30%, more preferably 5 to 20% by wt. of the composition and comprises:
a) at least one anionic surfactant;
b) at least one amphoteric and/or zwitterionic surfactant; and
c) optionally one or more nonionic surfactants, cationic surfactants, or blends thereof.

The anionic surfactant (which may comprise 2 to 40% by wt. of total composition) may be, for example, an aliphatic sulfonate, such as a primary alkane (e.g., C₈ - C₂₂) sulfonate, primary alkane (e.g., C₈ - C₂₂) disulfonate, C₈ - C₂₂ alkene sulfonate, C₈-C₂₂ hydroxyalkane sulfonate or alkyl glyceryl ether sulfonate (AGS); or an aromatic sulfonate such as alkyl benzene sulfonate, and the like.

The anionic may also be alkyl sulfosuccinates (including mono- and dialkyl, e.g., C₆-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, and acyl isethionates, and the like.

Sulfosuccinates may be monoalkyl sulfosuccinates having the formula:

R⁴O₂CCH₂CH(SO₃M)CO₂M

amido-MEA sulfosuccinates of the formula:

R⁴CONHCH₂CH₂O₂CCH₂CH(SO₃M)CO₂M

wherein R₄ ranges from C₈-C₂₂ alkyl and M is a solubilizing cation; amido-MIPA sulfosuccinates of formula:

RCONH(CH₂)CH(CH₃)SO₃M)CO₂M

where M is as defined above.

Also included are the alkoxylated citrate sulfosuccinates; and alkoxylated sulfosuccinates such as the following: 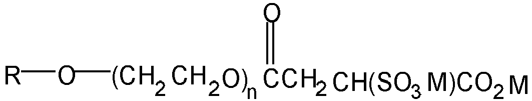 wherein n=1 to 20; and M is as defined above.

Sarcosinates are generally indicated by the formula RCON(CH₃)CH₂CO₂M, wherein R ranges from C₈ to C₂₀ alkyl and M is a solubilizing cation.

Taurates are generally identified by formula:

R²CONR³CH₂CH₂SO₃M

wherein R² ranges from C₈-C₂₀ alkyl, R³ ranges from C₁-C₄ alkyl and M is a solubilizing cation.

Another class of anionics are carboxylates such as follows:

R-(CH₂CH₂O)ₙCO₂M

wherein R is C₈ to C₂₀ alkyl; n is 0 to 20; and M is as defined above.

Another carboxylate which can be used is amido alkyl polypeptide carboxylates such as, for example, Monteine LCQ^{®} by Seppic.

Another surfactant which may be used are the C8-C18 acyl isethionates. These esters are prepared by reaction between alkali metal isethionate with mixed aliphatic fatty acids having from 6 to 18 carbons atoms and an iodine value of less than 20. At least 75% of the mixed fatty acids have from 12 to 18 carbons and up to 25% have from 6 to 10 carbon atoms.

Acyl isethionates, when present, will generally range from about 0.5-15% by weight of the total composition. Preferably, this component is present from about 1 to about 10%. The acyl isethionate may be an alkoxylated isethionate such as is described in US Patent No. 5,393,466, titled "Fatty Acid Esters of Polyalkoxylated Isethionic Acid" issued Feb. 28, 1995 to Ilardi et al. This compound has the general formula: 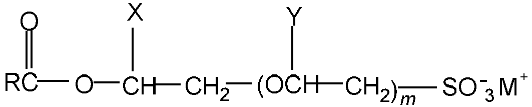 wherein R is an alkyl group having 8 to 18 carbons, m is an integer from 1 to 4, X and Y are hydrogen or an alkyl group having 1 to 4 carbons and M⁺ is a monovalent cation such as, for example, sodium, potassium or ammonium.

Another preferred class of anionics are N-acyl derivatives of amino acids. In the same preferred form, there is substantially no surfactant containing sulfate., preferably 0.1 or even 0.05% or less. Preferably sulfate containing surfactant is absent altogether. Preferred surfactants are acylglutamate, acylaspartate, acylglycinate and acylalaninate surfactants. Preferably, these are potassium and/or sodium salts of acylglutamate or acyl aspartate or acylglycinate or acylalaninate, wherein greater than 65% of the acyl chains has chain length C₁₄ or less, e.g., C₈ to C₁₄ (e.g., derived from coconut fatty acid). The acyl chains preferably have greater than 75%, more preferably greater than 80% C₁₄ or less chain length. Preferably, greater than 75%, most preferably greater than 80% of the chain length are C₁₂, C₁₄ or mixtures thereof.

There are two formats of amino acid surfactants commercially available. One is powder or flake format, which is typically more expensive and high in purity. Examples of solid dicarboxylic amino acid surfactants include:
- sodium N-cocoyl-L -glutamate (e.g., Amisoft^{®} CS-11 by Ajinomoto)
- sodium N-lauroyl-L- glutamate (e.g., Amisoft^{®} LS-11 by Ajinomoto)
- sodium N-myristoyl-L-glutamate (Amisoft^{®} MS-11 by Ajinomoto)
- potassium N-cocoacyl_I-Glutamate (e.g., Amisoft^{®} CK-11 by Ajinomoto)
- potassium N-myristoyl-L-glutamate (Amisoft^{®} MK-11 by Ajinomoto)
- potassium N-lauroyl-L-glutamate (Amisoft^{®} LK-11 by Ajinomoto)
- Sodium Lauroyl Aspartate (AminoFoamer^{™} FLMS-P1 by Asahi Kasei Chemical Corporation)
- Sodium Lauroyl Glutamate (Aminosurfact^{™} ALMS-P1/S1 by Asahi Kasei Chemical Corporation)
- Sodium Myristoyl Glutamate (Aminosurfact^{™} AMMS-P1/S1 by Asahi Kasei Chemical Corporation)

Examples of solid monocarboxylic amino acid surfactants include:
- sodium cocoyl glycinate (e.g., Amilite^{®} GCS-11 by Ajinomoto)
- potassium cocoyl glycinate (e.g., Amisoft^{®} GCK-11 by Ajinomoto)

Liquid amino acid surfactants typically contain 20-35% surfactant active, and are high in pH and inorganic salt (e.g. 3 to 6% NaCl). Examples include:
- AMISOFT^{®} ECS-22SB: Disodium Cocoyl Glutamate (30% Aqueous Solution)
- AMISOFT^{®} CS-22: Disodium Cocoyl Glutamate sodium Cocoyl Glutamate (25% Aqueous Solution)
- AMISOFT^{®} CK-22: Potassium Cocoyl Glutamate (30% Aqueous Solution)
- AMISOFT^{®} LT-12: TEA-Lauroyl Glutamate (30% Aqueous Solution)
- AMISOFT^{®} CT-12 TEA-Cocoyl Glutamate (30% Aqueous Solution)
- AMILlTE^{®} ACT-12: TEA-Cocoyl Alaninate (30% Aqueous Solution)
- AMILlTE^{®} ACS-12: Sodium Cocoyl Alaninate(30% Aqueous Solution)
- AMILlTE^{®} GCK-12/GCK-12K: Potassium Cocoyl Glycinate(30% Aqueous Solution)
- Aminosurfact^{™} ACDS-L: Sodium Cocoyl Glutamate (25% Aqueous Solution)
- Aminosurfact^{™} ACDP-L: Potassium Cocoyl Glutamate(22%)+Sodium Cocoyl Glutamate(7%)
- Aminosurfact^{™} ACMT-L: TEA-Cocoyl Glutamate(30% Aqueous Solution)
- AminoFoamer^{™} FLDS-L: Sodium Lauroyl Aspartate (25% Aqueous Solution)

### Zwitterionic and Amphoteric Surfactants

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. A general formula for these compounds is: 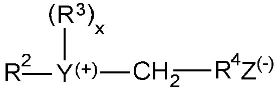 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms, R³ is an alkyl or monohydroxyalkyl group containing about 1 to about 3 carbon atoms; X is 1 when Y is a sulfur atom, and 2 when Y is a nitrogen or phosphorus atom; R⁴ is an alkylene or hydroxyalkylene of from about 1 to about 4 carbon atoms and Z is a radical selected from the group consisting of carboxylate, sulfonate, sulfate, phosphonate, and phosphate groups.

Examples of such surfactants include:
4-[N, N-di(2-hydroxyethyl)-N-octadecylammonio]-butane-1-carboxylate;
5-[S-3 hydroxypropyl-S-hexadecylsulfonio]-3-hydroxypentane-1-sulfate;
3-[P, P-diethyl-P-3,6,9-trioxatetradexocylphosphonio]-2-hydroxypropane-1-phosphate;
3-[N, N-dipropyl-N-3-dodecoxy-2-hydroxypropylammonio]-propane-1-phosphonate;
3-[N,N-diemthyl-N-hexadecylammonio)propoane-1-sulfonate;
3- [N,N-dimethyl-N-hexadecylammonio)-2-hydroxypropane-1-sulfonate;
4-[N, N-di(2-hydroxyethyl)-N-(2-hydroxydodecyl)ammonio]-butane-1-carboxylate;
3-[S-ethyl-S-(3-dodecoxy-2-hydroxypropyl)sulfonio]-[propane-1phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 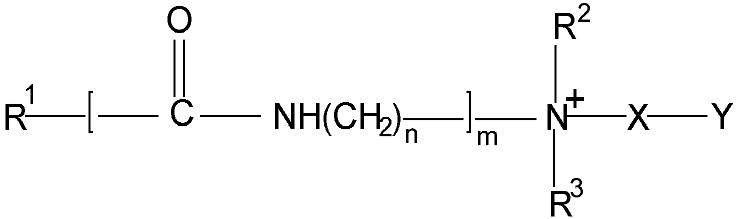
where R1 is alkyl or alkenyl of 7 to 18 carbon atoms;
R2 and R3 are each independently alkyl, hydroxyalkyl or caboxyalkyl of 1 to 3 carbon atoms;
n is 2 to 4;
m is 0 to 1;
X is alkylene of 1 to 3 carbon atoms optionally substituted with hydroxyl, and Y is -CO₂- or -SOs-

Suitable amphoteric detergents within the above general fomula include simple betaines of formula: 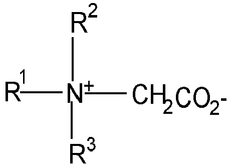 and amido betaines of formula: 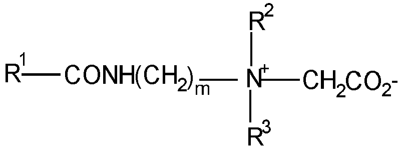 where m is 2 or 3.

In both, formulae R¹, R² and R³ are defined as previously. R¹ may in particular be a mixture of C₁₂ and C₁₄ alkyl groups derived from coconut so that at least half, preferably at least three quarters of the groups R¹ have 10 to 14 carbon atoms. R² and R³ are preferably methyl. A suitable betaine is cocoamidopropyl betaine.

A further possibility is that the amphoteric detergent is a sulphobetaine of formula: 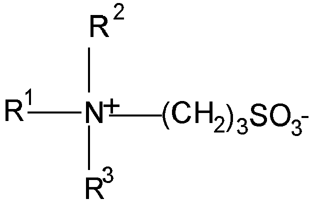 or 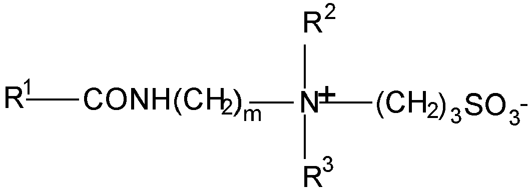 where m is 2 or 3, or variants of these in which 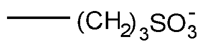 is replaced by

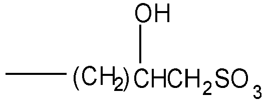

In these formulae R¹, R² and R³ are as discussed previously.

Amphoacetates and diamphoacetates are also intended to be covered in possible zwitterionic and/or amphoteric compounds which may be used, especially C8-C20 amphoacetates or mixtures thereof, and the like. A suitable amphoacetate is sodium laurylamphoacetate.

The amphoteric/zwitterionic surfactant, when used, generally comprises 0.1 to 30%, preferably 1 to 20% by weight,

A preferred surfactant system of the invention comprises the following: anionic surfactant (e.g., alkali metal alkyl ethersulfate) - 2-50%; amphoteric surfactant (e.g., alkyl betaine or alkyl amphoacetate) - 1-20%.

The surfactant system may also optionally comprise a nonionic surfactant. As noted, a characteristic of the invention is that, even if level of nonionic emulsifier is 0.4% or less, the composition still maintains lamellar structure.

The nonionic which may be used includes in particular the reaction products of compound shaving a hydrophobic group and a reactive hydrogen atom, for example aliphatic alcohols, acids, amides, or alkyl phenols with alkylene oxides, especially ethylene oxide either alone or with propylene oxide. Specific nonionic detergent compounds are alkyl (C₆-C₂₂) phenols-ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides, and the like.

The nonionic may also be a sugar amide, such as a polysaccharide amide. Specifically, the surfactant may be one of the lactobionamides described in US Patent No. 5,389,279 titled "Compositions comprising nonionic glycolipid surfactants" issued on Feb. 14, 1995 to Au et al. or it may be one of the sugar amides described in US Patent No. 5,009,814 titled "Use of n-polyhydroxyalkyl fatty acid amides as thickening agents for liquid aqueous surfactant systems" issued on Apr. 23, 1991 to Kelkenberg.

Other surfactants which may be used are described in US Patent No. 3,723,325 to Parran Jr. and alkyl polysaccharide nonionic surfactants as disclosed in US Patent No. 4,565,647 titled "Foaming surfactant compositions", issued on Jan. 21. 1986 to Llenado.

Preferred alkyl polysaccharides are alkylpolyglycosides of the formula:

R₂O(CₙH₂ₙO)ₜ(glycosyl)ₓ

wherein R₂ is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds,the alcohol or alkylpolyethoxy alcohol is formed first and then reacted with glucose, or a source of glycose, to form the glycoside (attachment at the 1-position). The additional glycosyl units can then be attached between their 1-position and the preceding glycosyl units 2-, 3-, 4- and/or 6-position, preferably predominantly the 2-position.

The nonionic may comprise 0 to 10% or 0 to 5% by wt. of the composition. However, in preferred embodiments, the non-ionic emulfiers of the type exemplified by Wei comprises 0.4% or less, preferably 0.3%, more preferably 0.2% or less and most preferably 0.1% or less by wt. of the composition. Unlike Wei reference discussed above, the lamellar structure remains stable even at these low levels of non-ionic emulsifier.

### Lamellar Structurant

The composition of the invention utilize about 0.1 to 20% by wt., preferably 1 to 10%, more preferably 2 to 5% by wt. of a structuring agent which works in the compositions to form a lamellar phase. Such lamellar phase enables the compositions to suspend particles more readily (e.g., emollient particles) while still maintaining good shear thinning properties. The lamellar phase also provides consumers with desired rheology ("heaping").

The structurant is preferably a fatty acid or ester derivative, thereof, a fatty alcohol, or trihydroxystearin, and the like. More preferably the structurant is selected from the group consisting of caprylic, capric, lauric acid, myristic acid and mixtures thereof.

Examples of fatty acids which may be used are C₈-C₂₂ acids such as the following: lauric acid, oleic acid, isostearic acid, linoleic acid, linolenic acid, ricinoleic acid, elaidic acid, arachidonic acid, myristoleic acid and palmitoleic acid, and the like. Ester derivatives include propylene glycol isostearate, propylene glycol oleate, glyceryl isostearate, glyceryl oleate and polyglyceryl diisostearate, and the like.

### Moisturizing and conditioning agent (e.g., oil/emollient)

One of the principle benefits of the invention is the ability to suspend oil/emollient particles in a lamellar phase composition (and ability to do so in presence of low amounts of non-ioinc. The following oil/emollients, for example, may optionally be suspended in the compositions of the invention.

Various classes of oils are set forth below.

Vegetable oils: arachis oil, castor oil, cocoa butter, coconut oil, corn oil, cotton seed oil, olive oil, palm kernel oil, rapeseed oil, safflfower seed oil, sesame seed oil, sunflower seed oil and soybean oil, and the like.

Esters: butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate, and the like.

Animal fats: acetylated lanolin alcohols, lanolin, lard, mink oil and tallow, and the like.

Other examples of oil emollients includes mineral oil, petrolatum, silicone oil such as dimethyl polysiloxane, lauryl and myristyl lactate, and the like.

The emollient/oil is generally used in an amount from about 1 to 25%, preferably 1 to 10% by wt. of the composition.

### Cationic Conditioner

The composition comprises 0.01 to 5%, preferably 0.1 to 2% by wt. of a cationic deposition polymer (cationic conditioner).

Cationic conditoners may include Quatrisoft^{®} LM-200, polyquaternium-24, Merquat^{®} plus 3330, polyquaternium-39 and Jaguar^{®} type polymers. They may further include cationic cellulose type polymers such as Polyquaternium 10 (UCare Series from Dow) and synthetic cationic polymers such as Polyquaternium 49 and polyquaternium 51.

### (Merquat Series from Lubrizol)]

Cationic deposition polymers may also include hydrophobically modified cationic cellulose polymers such as SoftCat Series from Dow Chemical Company.

In addition, the compositions of the invention may include optional ingredients as follows: Organic solvents, such as ethanol; auxiliary thickeners, sequestering agents, such as tetrasodium ethylenediamine-tetraacetate (EDTA), EHDP or mixtures in an amount of 0.01 to 1%, preferably 0.01 to 0.05%; and coloring agents, opacifiers and pearlizers such as zinc stearate, magnesium stearate, TiO₂, or EGMS (ethylene glycol monostearate); all of which are useful in enhancing the appearance of cosmetic properties of the product.

The compositions may further comprise antimicrobials such as 2-hydroxy4,2'4' trichlorodiphenylether (DP300); preservatives such as dimethyloldimethylhydantoin (Glydant XL1000), parabens, sorbic acid, etc.

The compositions may also comprise coconut acyl mono- or diethanol amides as suds boosters.

Antioxidants such as, for example, butylated hydroxytoluene (BHT) may be used advantageously in amounts of about 0.01% or higher if appropriate.

Another optional ingredient which may be added are the deflocculating polymers such as are taught in US Patent No. 5,147,576 title "Liquid Detergent Composition in the Form of Lamellar Deoplets Containing a Deflocculating Polymer", issued on Sept. 15, 1992 to Montague. Other ingredients which may be included are exfoliants such as polyoxyethylene beads, walnut sheets and apricot seeds, and the like.

The compositions of the invention, as noted, are lamellar compositions. In particular, the lamellar phase comprises 20 to 80%, preferably 30 to 65% of the total phase volume. The phase volume may be measured, for example, by conductivity measurements or other measurements which are well known to those skilled in the art. While not wishing to be bound by theory, higher phase volume is believed to provide better suspension of emollients.

The structuring polymers of the invention are hydrophobically modified anionic cross-linked polymers. Such polymers are also referred to as "associative" polymers.

These polymers are constituted by a hydrophilic main chain and hydrophobic side-chains. Their behaviour in solution is a result of competition between the hydrophobic and hydrophilic properties of their structure. The hydrophobic units lead to the formation of aggregates constituting linkage points between the macromolecular chains. From a rheological viewpoint, these generally water-soluble (e.g. typically soluble under slightly acidic or alkaline conditions) polymers have a very high viscosifying power in water and retain their viscosity well in a saline medium. In mixed polymer and surfactant systems, surfactant aggregates can form, which are stabilized by different types of interactions: electrostatic interactions, dipolar interactions, or hydrogen bonds. The typically water-soluble polymers can interact more specifically with surfactants due to their hydrophobic portions.

The hydrophilic main chain of these polymers can, in particular, result from polymerization of a hydrophilic monomer containing functions onto which hydrophobic chains can subsequently be grafted, for example acid functions. This method of preparing the polymers is described in particular in the "Water Soluble Polymers", ACS Symposium Series 467, ed. Shalaby W. Shalaby et al., Am. Chem. Soc. Washington (1991), pp. 82-200. However, a water-soluble polymer of natural origin, or a natural polymer rendered water-soluble by chemical modification, can also be used. Polymers can also be formed by copolymerization of hydrophilic monomers and hydrophobic monomers. These hydrophobic polymers, introduced into the reaction medium in a much smaller quantity than the hydrophilic polymers, generally comprise a fatty hydrocarbon chain. This method of preparation is described in the publication by S. Biggs et al., J. Phys. Chem. (1992, 96, pp 1505-11).

Examples of these polymers are acrylic polymers, polyethers, and polyosidic chains which may be partially substituted. The hydrophilic main chain is constituted as monomers carrying highly hydrophobic pendant groups. The molar percentage of monomers carrying hydrophobic pendant groups is termed the modification percentage of the hydrophilic chain. The hydrophobic pendant groups can be any hydrophobic pendant group which is conventionally used to prepare the polymers. In one aspect, the hydrophobic groups used comprise a backbone containing at least 8 carbon atoms, preferably 10 to 28 carbon atoms.

Particular examples of these hydrophobic groups are linear, branched, saturated or unsaturated hydrocarbon chains which may or may not contain cycles. Preferred examples of hydrophobic groups are hydrocarbon chains, in particular alkyl chains, containing 8 to 28 carbon atoms, preferably 12 to 22 carbon atoms. Modified units are advantageously in the form of an ether, ester or amide. This is particularly the case when the main chain of the associative polymer is an acrylic chain. The associative polymers used can have a mass average molar mass in the range 10⁴ to 10⁷.

The concentration of polymer in the composition is generally in the range about 0.01% to about 5 or from about 0.05 to about 2.0% by weight, preferably from about 0.1% to about 1.0% by weight or from about 0.1% to about 0.5% by weight of the composition. Preferred associative polymers include hydrophobically modified polyacrylates; hydrophobically modified polysaccharides; hydrophobically modified urethanes. Nonlimiting examples of polymers include Acrylates/Vinyl Isodecanoate Crosspolymer (Stabylen 30 from 3V), Acrylates/C 10-30 Alkyl Acrylate Crosspolymer (Pemulen TR1 and TR2), Ammonium Acryloyldimethylaurate/Beheneth-25 Methacrylate Crosspolymer (Aristoflex HMB from Clariant), Arylates/Beheneth-25 Methacrylate Copolymer (Aculyn 28 from Rohm and Haas)); Acrylates/Steareth-20 Methacrylate Copolymer (Aculyn 22 from Rohm and Haas), PEG-150/Decyl Alclhol/SMDI Copolymer (Aculyn 44 from Rohm and Haas), PEG-150 Distearate (Aculyn 60 from Rohm and Haas), Acrylates/Steareth-20 Methacrylate Crosspolymer (Aculyn 88 from Rohm and Haas).

In one embodiment, the polymer is a crosslinked alkali swellable, associative polymer comprising acidic monomers and associative monomers with hydrophobic end groups, where the polymer comprises a percentage hydrophobic modification and a hydrophobic side chain comprising alkyl functional groups having a length. Without intending to be limited by theory, it is believed that the acidic monomers contribute to the ability of the polymer to swell in water upon neutralization of the acidic groups; and associative monomers (e.g. monomers with hydrophobic chains) anchor the polymer into structured surfactant hydrophobic domains, e.g., lamellae, to confer structure to the surfactant compositions and keep the polymer from collapsing and losing effectiveness in the presence of electrolyte. The crosslinked associative polymer comprises a percentage hydrophobic modification, which is the mole percentage of monomers expressed as a percentage of the total number of all monomers in the polymer backbone, including both acidic and other non-acidic monomers. The percentage hydrophobic modification of the polymer, hereafter % HM can be determined by the ratio of monomers added during synthesis, or by analytical techniques such as proton nuclear magnetic resonance (NMR). The alkyl side chain length can be determined similarly. Monomers comprising only 2 or fewer alkyl hydrocarbons (e.g., ethyl, methyl) are not considered associative for the purposes of the present invention, all side chains having more than 2 carbons being associative. Associative alkyl side chains comprise for example butyl, propyl, stearyl, steareth, cetyl, lauryl, laureth, octyl, behenyl, beheneth, steareth, or other linear, branched, saturated or unsaturated alkyl or alketh hydrocarbon side chains.

Preferred polymers of the invention are hydrophobically or non-hydrophobically modified crosslinked polyacrylates rather than hydrophobically modified linear polyacrylates. Their viscosity is typically much lower.

More specifically, viscosity of a 2% solution (e.g. in water), (measured at pH 6.35 and shear rate 5S⁻¹ , and further measured at 25degC using a rheometer), is in range of 1 to 100 mPa.s (centipoise), more preferably 10 to 80 mPa.s (centipoise) and most preferably 20 to 60 mPa.s (centipoise). Moreover, slope of the viscosity shear rate curve is in the range of -0.6 to - 1.2, more preferably in the range of -0.7 to -1.1 and most preferably in the range of -0.8 to -1.0.

The slope measures the shear thinning nature of the polymer, wherein the higher the slope (more negative), the more shear thinning the polymer is.

Composition of the invention contain 0.4% or less, preferably 0.3% or less, more preferably 0.2% or less, most preferably 0.1% or less of HLB 1.4-13 nonionic emulsifiers such as those used in US Patent Nos. 8,105, 996 and Us 8,158,566 to Wei et al noted above.

Further compositions of the invention comprise 2.7% or less, preferably 2.5% or less and more preferably 2% or less of salt, especially sodium chloride.

### Examples

The following compositions were prepared. The petrolatum emulsion was prepared as noted in Table 2 and in the explanation of preparation set forth below Table 2.

**Table 1**

| Formula Summary: | | | | | |
|---|---|---|---|---|---|
| | Example 1 | Comp. Example 2 | Example 3 | Comp. Example 4 | Comparative Example A |
| Chemical/Trade Name | | | | | |
| Water | Qs (sufficient to 100%) | Qs | qs | qs | qs |
| Starch hydroxyl propyl phosphate | 1.20 | 1.20 | 1.20 | 1.20 | 1.20 |
| Sodium laureth-1 sulfate | | | | | 13.00 |
| Sodium trideceth-3 sulfate | | | | 13.00 | |
| Sodium cocoyl isethionate | 5.00 | 5.00 | 5.00 | | |
| Sodium lauroyl glycinate | 3.33 | 3.33 | 3 | | |
| Sodium methyl acyl taurate | 5.00 | 5.00 | | | |
| Sodium lauroyl glutamate | | | 5.00 | | |
| Sodium lauramphoacetate | 1.67 | 1.67 | - | | |
| Sodium cocoamidopropyl betaine | | | 2 | 2.00 | 2.00 |
| Lauric acid | 3.00 | 3.00 | 3.00 | 3.00 | 3.00 |
| Soybean oil | 1.20 | 1.20 | 1.20 | 1.20 | 1.20 |
| Fully hydrogenated soy bean oil | 1.80 | 1.80 | 1.80 | 1.80 | 1.80 |
| Stearic Acid | 0.25 | 0.25 | 0.25 | 0.25 | 0.25 |
| BHT | 0.10 | 0.10 | 0.10 | 0.10 | 0.10 |
| Structuring Polymer* | Y | Y | Y | Y | Y |
| Glycerin | 1.00 | 1.00 | 1.00 | 1.00 | 1.00 |
| Guar hydroxypropyl trimonium chloride, Jaguar C14 (Solvay Chemicals) | 0.40 | | 0.40 | 0.40 | 0.40 |
| Cationic Cellulose, Polymer JR (Dow Chemicals) | | 0.40 | | | |
| Petrolatum emulsion** | 8.00 | 8.00 | 8.00 | 8.00 | 8.00 |
| | | | | | |
| Glydant Plus Liquid | 0.35 | 0.35 | 0.35 | 0.35 | 0.35 |
| EDTA (Versene XL 100) | 0.05 | 0.05 | 0.05 | 0.05 | 0.05 |
| Citric Acid | 0.09 | 0.09 | 0.09 | 0.09 | 0.09 |
| NaOH (to titrate to pH) | 6.6 +/-0.3 | 6.6. +/-0.3 | 6.6+/- 0.3 | 6.6 +/-0.3 | 6.6 +/- 0.30 |
| Fragrance | 1.30 | 1.30 | 1.30 | 1.30 | 1.30 |
| Total | 100.00 | 100.00 | 100.00 | 100.00 | 100.00 |

| | | | | | |
|---|---|---|---|---|---|
| * Various structuring polymers were used and their concentration varied, in the range of 0.2 to 1.05%, to obtain a similar formulation TA viscosity, as measured by Brookfield viscometer, in the range 70,000 -110,000 mPa.s (cP). ** Composition of Petrolatum emulsion and procedure used in making the emulsion are described below. | | | | | |

**Table 2**

| Typical Composition of Petrolatum Emulsion and Procedure for making the emulsion | |
|---|---|
| Component | Wt. % |
| Sodium cocoyl isethionate | 6.0 |
| Lauric acid | 3.5 |
| Petrolatum | 55.0 |
| Water | To 100 |
| Glydant Plus Liquid | 0.158 |

| Procedure: | |
|---|---|
| Ingredient | Wt. % |

| Oil Phase: | |
|---|---|
| G-2212 Petrolatum | 55.00 |
| Lauric Acid | 3.50 |

| Aqueous Phase: | |
|---|---|
| Enriched SCI (85%) | 6.000 |
| DI water | 35.342 |
| Glydant plus liquid | 0.158 |
| Total | 100.000 |

1. The aqueous phase was mixed and heated in a stirred tank mixer, and oil phase was heated in a separate beaker. Once heated, the oil phase was added to the aqueous phase containing stirred tank mixer. Both oil phase and aqueous phases were heated to 75 degrees C, either separately and combined, or after they have been combined. After the phases were combined and completely covered, the homogenizer was turned up to 1000rpm.
2. After addition of the phases, the composition was mixed and homogenized for 10 minutes at 4000 rpm
3. The compositions was passed (one pass) through a nano DeBee, a bench scale sonolator, at 5000 psi.

**Table 3**

| Mildness of different formulations measured using Corneosurfametry (Ref. Liu et al., Journal of Cosmetic Science 2016, 38, 178-186) | |
|---|---|
| Means Comparisons for CIM- Comparisons for all pairs using Tukey-Kramer HSD (95% confidence level) | |
| | CIM Mean |
| | |
| Example 3 | 57.65* |
| Comp. Ex. 4 | 57.12* |
| Example 1 | 56.70* |
| Comparative A | 50.38** |

Values followed by *are not significantly different from each other (Examples 1, 2 and 3), while that followed by ** (Comparative A) is significantly different from those followed by * (again Examples 1,2, and 3)

Table 3 above demonstrates that mildness of composition can vary depending, for example, on surfactant composition.

**Table 4**

| Amount of Petrolatum Deposited in grams and viscosity of 2% polymer solution @pH 6.35 and shear rate of 5s-1 | | | | | |
|---|---|---|---|---|---|
| Structuring Polymer | INCI Name | Supplier | Amount of Petrolatum Deposited in grams | Viscosity in mPa.(cP) @ 5s-1 of 2% polymer solution at pH 6.35 (M) | Slope of the viscosity shear rate curve |
| | | | | | From .05 to 1000 S⁻¹ |
| Hydrophobically modified cross linked polyacrylates ↓ | | | | | |
| Aculyn 88 | Acrylates/ Steareth-20 methacrylates cross polymer | Dow Chemicals | 0.25 | 33.7 | -0.84 |
| Aqupec SER W300C | Acrylates/ C10-30 alkyl acrylate cross polymer | Sumitomo Seika Chemicals | 0.21 | 51.7 | -0.85 |
| Pemulen TR1 | Acrylates/ C10-30 alkyl acrylate cross polymer | Lubrizol | 0.21 | 52.0 | -0.90 |
| Pemulen TR2 | Acrylates/ C10-30 alkyl acrylate cross polymer | Lubrizol | 0.15 | 67.0 | -0.97 |
| Ultrez 20 | Acrylates/ C10-30 alkyl acrylate cross polymer | Lubrizol | 0.18 | 66.0 | -0.99 |
| Stabylen 30 | Acrylates/ vinyl isodecanoate cross polymer | 3V Inc. | 0.13 | 65.1 | -0.97 |

| Non-hydrophobically modified cross-linked polyacrylate | | | | | |
|---|---|---|---|---|---|
| Carbapol 980 | Carbomer | Lubrizol | 0.18 | 53.8 | -0.8 |
| | | | | | |

| Hydrophobically modified linear polyacrylates | | | | | |
|---|---|---|---|---|---|
| Synthalen W2000 | Acrylates/ palmeth 25 acrylate copolymer | 3V Inc. | 0.09 | 111.0 | -0.79 |
| Aculyn 28 | Acrylates/ beheneth 25 methacrylate copolymer | Dow Chemicals | 0.06 | 119.6 | -1.10 |

### Discussion

From table 4, it can be seen that deposition is a function of the type of structuring polymer used.

More specifically, it is seen from Table 4 that deposition is inversely proportional to the viscosity of the 2% polymer solution measured at @ pH 6.35 and a shear rate of 5s-1. The relationship is linear with a confidence level of over 90%, irrespective of whether the hydrophobic polymer is linear or cross linked. It is also seen that crosslinked polymers, hydrophobically or non-hydrophobically modified, deposit much higher levels of petrolatum than hydrophobically modified linear polymers.

The procedure used for determining petrolatum deposition was as follows:
An 8x8 inch piece of silk was washed with 12 ml of body wash base formula containing no PJ emulsion. A blank deposition value was obtained by subtracting the weight of the untreated silk piece from the weight of the washed and dried silk. Next, another silk with body wash containing PJ emulsion was washed. Overall PJ deposition is measured as the weight of silk after washing and drying minus the initial weight of the silk minus the blank deposition.

**Table 5**

| Amount of Polymer Deposited (D) from Various formulations in grams | | | | | | |
|---|---|---|---|---|---|---|
| Structuring Polymer | INCI Name | Example 1 | Example 2 | Example 3 | Comp. Ex. 4 | Comparative Example A |
| No polymer | - | 0.39 | | | | |
| Aculyn 88 | Acrylates/ Steareth-20 methacrylates cross polymer | 0.25 | | | | |
| Aqupec SER W300C | Acrylates/ C₁₀₋₃₀ alkyl acrylate cross polymer | 0.21 | | | | |
| Pemulen TR1 | Acrylates/ C₁₀₋₃₀ alkyl acrylate cross polymer | 0.21 | 0.16 | 0.25 | 0.18 | 0.13 |
| Pemulen TR2 | Acrylates/ C₁₀₋₃₀ alkyl acrylate cross polymer | 0.15 | | | | |
| Ultrez 20 | Acrylates/ C₁₀₋₃₀ alkyl acrylate cross polymer | 0.18 | | | | |
| Stabylen 30 | Acrylates/ vinyl isodecanoate cross polymer | 0.13 | | | | |
| Carbopol 980 | Carbomer | 0.18 | | | | |
| Synthalen W2000 | Acrylates/ palmeth 25 acrylate copolymer | 0.09 | | | | 0.14 |
| Aculyn 28 | Acrylates/ beheneth 25 methacrylate copolymer | 0.06 | 0.03 | 0.01 | 0.05 | 0.14 |

Table 5 shows that the deposition depends on the polymer used for those formulations of Examples 1 through 3 which statistically have the same level of mildness (see Table 3). By contrast, in Formulation of Comparative Example 4, a formulation which is statistically harsher than the other formulations, deposition is not affected by the polymer used. This demonstrates that the effect on deposition is a function of mildness as defined by CIM value. The polymers of lower viscosity (e.g., Pemulen TR1 versus Aculyn 88) enhance deposition of oil when composition has CIM value, for example, 55 or greater, but do not enhance viscosity when composition has CIM value below 55.

**Table 6**

| Inventive Examples 1 and 2 on Table 3 from US 8,158,566 B2 at different levels of nonionic emulsifier Trideceth-3 and salt with Stabelyn 30 and Aculyn 28 as the associative polymer following the procedure taught in the patent | | | | | |
|---|---|---|---|---|---|
| Associative Polymer | Associative Polymer Level, % | Trideceth-3 level, % | Sodium chloride level, % | Youngs Modulus (Pa) | Youngs Modulus (Pa) from US 8,158,566 B2 |
| None | 0 (Patent Example - A-3 Table 3) | 1.4 | 3.3 | 22.4 | 90 |
| Aculyn 28 | 1.0 | 0.1 | 3.3 | 1.8 | - |
| Aculyn 28 | 1.0 | 0.35 | 3.3 | 3.3 | - |
| Aculym 28 | 1.0 (Patent Example - 2 Table 3) | 1.4 | 3.3 | 68.5 | 288 |
| Stabelyn 30 | 1.0 | 0.35 | 3.3 | 55.9 | - |
| * Stabelyn 30 | 1.0 | 0.35 | 2.7 | 8.2 | - |

| | | | | | |
|---|---|---|---|---|---|
| * This sample became unstable after two weeks showing a distinct separation in to two phases | | | | | |

The inventive example #2 on Table 3 in US 8,158,566 B2 using Aculyn 28 as the associative polymer was reproduced following the procedure enumerated in the patent at different levels of Trideceth-3 (non-ionic emulsifier). Also, comparative Example A-3 was reproduced. The results are shown in Table 6. The results show that the Youngs Modulus applicants obtained without the associative polymer is 22.4 Pa compared to the value 90 Pa indicated in the patent. In other words, their Young's Modulus of 90 approximately equals our Youngs Modulus of 22.4 and the scaling factor thus would be 90 divided by 24 which equals 4.1. Therefore, we can show that anything below approximately 24.4 Pa in our example will be below the corresponding target value of 100 Pa indicated in the footnote of Table 3 in US 8,158,566 B2, that is 100 divided by scaling factor 4.1 equals 24.4 Anything under such 100 Pa value (equivalent to 24.4 Pa in our reproduced experiments) fails because stability is compromised and composition is therefore undesirable.

The inventive Example 1 on Table 3 in US 8,158,566 B2 using Stabylen as the associative polymer was reproduced following the procedure enumerated in the patent at 0.35% Trideceth-3 (non-ionic emulsifier) at two different salt levels of 3.3% and 2.7%. These results show that at 0.35% non-ionic emulsifier and a salt level of 2.7% the Youngs Modulus falls well below the threshhold value of (24.4) Pa (which is equivalent to 100Pa in Wei patent). Also, the sample at 0.35% non-ionic emulsifier and a salt level of 2.7% was unstable and showed complete phase separation after two weeks.

Based on these results, below a non-ionic, Trideceth-3, level of 0.4%, in particular 0.35%, and a salt, sodium chloride, level of 2.7% define the thresh hold value of Youngs Modulus.

## Claims

1. A lamellar composition comprising:
a) 1 to 70%, preferably 2 to 30% and more preferably 5 to 20% of a mixture of anionic surfactant comprising acylglutamate, acylglycinate, acyl isethionate and/or methylacyltaurate; amphoteric comprising amphoacetate and /or zwitterionic surfactants comprising cocoamidopropyl betaine and/or sulphobetaine; as well as optional nonionic and other surfactants;
b) 0.1 to 20%, preferably 1 to 10% and more preferably 2 to 5 wt. % of a Csto C₁₄ fatty acid, such as caprylic, capric, lauric acid, myristic acid or mixtures thereof;
c) 0.1 to 25%, preferably 1 to 15% and more preferably 2 to 10% of a moisturizing, conditioning agent, such as emulsion of petrolatum and/ or triglyceride oil;
d) 0.01 to 5%, preferably 0.1 to 2% and more preferably 0.2 to 1% of a cationic deposition polymer (also may be referred to a cationic conditioning polymer) comprising guar hydroxypropyl trimonium chloride;
e) 0.01 to 5%, preferably 0.05 to 2% or 0.1 to 1% of a hydrophobically or non-hydrophobically modified anionic cross linked polymer with the said polymer(s) having a viscosity of 2% solution, when measured at pH 6.35 and shear rate of 5s⁻¹, in the range of 1 to 100 mPa.s (centipoise) more preferably 10 to 80 mPa.s (centipoise) and most preferably in the range of 20 to 60 mPa.s (centipoise); and wherein the slope of the viscosity shear rate curve is in the range of - 0.6 to -1.2, more preferably in the range of - 0.7 to - 1.1 and most preferably in the range of - 0.8 to - 1.0;
f) 0.1 to 20% by wt. lamellar structurant;
and
wherein the cleansing formulation has level of nonionic emulsifiers of HLB 1.4-13, 0 to 0.4%, preferably 0 or 0.1 to 0.35%, preferably 0.3% or less or 0.25% or less, and 2.7% or less, preferably 2.5% or less and more preferably 2% or less of salt, especially sodium chloride,the composition being substantially free of sulfate-containing surfactant.

2. A composition according to claim 1, wherein the composition has a corneosurfametry Calorimetric Index of Mildness, CIM, as defined in Liu et al., Journal of Cosmetic Science 2016, 38, 178-186, of 53 or greater, preferably 55 or greater and more preferably 55 to 65.

3. A composition according to claim 1 or 2, wherein the moisturizing/condisioning agent is a sub-micron Petrolatum emulsion.

4. A composition according to any one of claims 1 to 3, wherein the moisturizing/conditioning agent is a triglyceride oil.

5. A composition according to claim 4, wherein the triglyceride oil is sunflower seed oil or soybean oil.

6. A composition according to any one of claims 1 to 5, wherein the cationic deposition polymer is guar hydroxypropyl trimonium chloride.

7. A composition according to any one of claims 1 to 6, wherein the cationic deposition polymer is a cationically substituted cellulose derivative.

8. A composition according to any one of claims 1 to 7, wherein the hydrophobically or non hydrophobically modified polymer is carbomer, Acrylates/ Steareth-20 methacrylates cross polymer, Acrylates/ C10-30 alkyl acrylate cross polymer, or Acrylates/ vinyl isodecanoate cross polymer.

9. A composition according to any one of claims 1 to 8, in which nonionic surfactant is present and is CMEA.

## Patentansprüche

1. Lamellare Zusammensetzung, umfassend:
a) 1 bis 70%, vorzugsweise 2 bis 30% und bevorzugter 5 bis 20% einer Mischung aus anionischem Tensid, umfassend Acylglutamat, Acylglycinat, Acylisethionat und/oder Methylacyltaurat, amphoterem Tensid, umfassend Amphoacetat, und/oder zwitterionischen Tensiden, umfassend Cocoamidopropylbetain und/oder Sulfobetain, sowie optional nichtionischen und anderen Tensiden;
b) 0,1 bis 20%, vorzugsweise 1 bis 10% und bevorzugter 2 bis 5 Gew.-% einer C₈- bis C₁₄-Fettsäure, wie Caprylsäure, Caprinsäure, Laurinsäure, Myristinsäure oder Mischungen davon;
c) 0,1 bis 25%, vorzugsweise 1 bis 15% und bevorzugter 2 bis 10% eines feuchtigkeitsspendenden Konditionierungsmittels, wie beispielsweise eine Emulsion von Vaseline und/oder Triglyceridöl;
d) 0,01 bis 5%, vorzugsweise 0,1 bis 2% und bevorzugter 0,2 bis 1% eines kationischen Abscheidungspolymers (kann auch als kationisches Konditionierungspolymer bezeichnet werden), umfassend Guarhydroxypropyltrimoniumchlorid;
e) 0,01 bis 5%, vorzugsweise 0,05 bis 2% oder 0,1 bis 1% eines hydrophob oder nicht-hydrophob modifizierten anionischen vernetzten Polymers, wobei das (die) Polymer(e) in 2%-iger Lösung, wenn bei einem pH-Wert von 6,35 und einer Scherrate von 5 s⁻¹ gemessen wird, eine Viskosität in dem Bereich von 1 bis 100 mPa.s (centipoise), bevorzugter 10 bis 80 mPa.s (centipoise) und höchst bevorzugt in dem Bereich von 20 bis 60 mPa.s (centipoise) aufweisen und wobei die Steigung der Kurve der Viskositätsscherrate in dem Bereich von -0,6 bis -1,2, bevorzugter in dem Bereich von -0,7 bis -1,1 und höchst bevorzugt in dem Bereich von -0,8 bis -1,0 liegt;
f) 0,1 bis 20 Gew.-% lamellares Strukturierungsmittel; und
wobei die Reinigungsformulierung einen Gehalt an nicht-ionischen Emulgatoren eines HLB-Wertes von 1,4-13 von 0 bis 0,4%, vorzugsweise 0 oder 0,1 bis 0,35%, vorzugsweise 0,3% oder weniger oder 0,25% oder weniger aufweist und 2,7% oder weniger, vorzugsweise 2,5% oder weniger und bevorzugter 2% oder weniger Salz, insbesondere Natriumchlorid, aufweist, wobei die Zusammensetzung im Wesentlichen frei von sulfathaltigem Tensid ist.

2. Zusammensetzung nach Anspruch 1, wobei die Zusammensetzung einen Corneosurfametrie-Kalorimetrie-Index der Mildheit, CIM, wie in Liu et al., Journal of Cosmetic Science 2016, 38, 178-186 definiert, von 53 oder mehr, vorzugsweise von 55 oder mehr und bevorzugter von 55 bis 65, aufweist.

3. Zusammensetzung nach Anspruch 1 oder 2, wobei das feuchtigkeitsspendende/ konditionierende Mittel eine Vaseline-Emulsion im Submikron-Bereich ist.

4. Zusammensetzung nach irgendeinem der Ansprüche 1 bis 3, wobei das feuchtigkeitsspendende/ konditionierende Mittel ein Triglyceridöl ist.

5. Zusammensetzung nach Anspruch 4, wobei das Triglyceridöl Sonnenblumenöl oder Sojaöl ist.

6. Zusammensetzung nach irgendeinem der Ansprüche 1 bis 5, wobei das kationische Abscheidungspolymer Guarhydroxypropyltrimoniumchlorid ist.

7. Zusammensetzung nach irgendeinem der Ansprüche 1 bis 6, wobei das kationische Abscheidungspolymer ein kationisch substituiertes Cellulosederivat ist.

8. Zusammensetzung nach irgendeinem der Ansprüche 1 bis 7, wobei das hydrophob oder nicht-hydrophob modifizierte Polymer Carbomer, Acrylat/Steareth-20-Methacylat-Kreuzpolymer, Acrylat/C₁₀₋₃₀-Alkylacrylat-Kreuzpolymer oder Acrylat/ Vinylisodecanoat-Kreuzpolymer ist.

9. Zusammensetzung nach irgendeinem der Ansprüche 1 bis 8, worin nichtionisches Tensid vorliegt und CMEA ist.

## Revendications

1. Composition lamellaire comprenant :
a) 1 à 70 %, de préférence 2 à 30 % et de manière davantage préférée 5 à 20 % d'un mélange de tensioactif anionique comprenant de l'acylglutamate, de l'acylglycinate, de l'iséthionate d'acyle et/ou du méthylacyltaurate ; de tensioactifs amphotères comprenant de l'amphoacétate et/ou de tensioactifs zwitterioniques comprenant de la cocoamidopropyl bétaïne et/ou de la sulfobétaïne ; ainsi que des tensioactifs facultatifs non ioniques et autres ;
b) 0,1 à 20 %, de préférence 1 à 10 % et de manière davantage préférée 2 à 5 % en poids d'un acide gras en C₈ à C₁₄, tel que l'acide caprylique, caprique, laurique, myristique ou des mélanges de ceux-ci ;
c) 0,1 à 25 %, de préférence 1 à 15 % et de manière davantage préférée 2 à 10 % d'un agent conditionnant hydratant, tel qu'une émulsion de pétrolatum et/ou d'huile de triglycéride ;
d) 0,01 à 5 %, de préférence 0,1 à 2 % et de manière davantage préférée 0,2 à 1 % d'un polymère de dépôt cationique (pouvant également être appelé polymère de conditionnement cationique) comprenant du chlorure d'hydroxypropyl trimonium de guar ;
e) 0,01 à 5 %, de préférence 0,05 à 2 % ou 0,1 à 1 % d'un polymère réticulé anionique modifié de manière hydrophobe ou non hydrophobe, ledit ou lesdits polymères ayant une viscosité de solution à 2 %, lorsqu'elle est mesurée au pH 6,35 et à un taux de cisaillement de 5 s⁻¹, dans la plage de 1 à 100 mPa.s (centipoise), de manière davantage préférée de 10 à 80 mPa.s (centipoise) et de manière préférée entre toutes dans la plage de 20 à 60 mPa.s (centipoise) ;
et dans laquelle la pente de la courbe viscosité taux de cisaillement est dans la plage de -0,6 à -1,2, de manière davantage préférée dans la plage de -0,7 à -1,1 et de manière préférée entre toutes dans la plage de -0,8 à - 1,0 ;
f) 0,1 à 20 % en poids de structurant lamellaire ;
et
dans laquelle la formulation de nettoyage a un niveau d'émulsifiants non ioniques de HLB 1,4-13, de 0 à 0,4 %, de préférence de 0 ou 0,1 à 0,35 %, de préférence de 0,3 % ou moins ou de 0,25 % ou moins, et de 2,7 % ou moins, de préférence de 2,5 % ou moins et de manière davantage préférée de 2 % ou moins de sel, notamment de chlorure de sodium, la composition étant sensiblement exempte de tensioactif contenant du sulfate.

2. Composition selon la revendication 1, dans laquelle la composition a un indice calorimétrique de douceur, CIM, par cornéosurfamétrie, tel que défini dans Liu et al., Journal of Cosmetic Science 2016, 38, 178-186, de 53 ou plus, de préférence de 55 ou plus et de manière davantage préférée de 55 à 65.

3. Composition selon la revendication 1 ou 2, dans laquelle l'agent hydratant/conditionnant est une émulsion de pétrolatum submicronique.

4. Composition selon l'une quelconque des revendications 1 à 3, dans laquelle l'agent hydratant/conditionnant est une huile de triglycéride.

5. Composition selon la revendication 4, dans laquelle l'huile de triglycéride est de l'huile de graines de tournesol ou de l'huile de soja.

6. Composition selon l'une quelconque des revendications 1 à 5, dans laquelle le polymère de dépôt cationique est du chlorure d'hydroxypropyl trimonium de guar.

7. Composition selon l'une quelconque des revendications 1 à 6, dans laquelle le polymère de dépôt cationique est un dérivé de cellulose à substitution cationique.

8. Composition selon l'une quelconque des revendications 1 à 7, dans laquelle le polymère modifié de manière hydrophobe ou non hydrophobe est un carbomère, un polymère réticulé d'acrylates/méthacrylates de stéareth-20, un polymère réticulé d'acrylates/acrylate d'alkyle en C10-30, ou un polymère réticulé d'acrylates/isodécanoate de vinyle.

9. Composition selon l'une quelconque des revendications 1 à 8, dans laquelle le tensioactif non ionique est présent et est du CMEA.
